# EUROPEAN PATENT APPLICATION

(11) **EP 2 060 263 A1**
(43) Date of publication of application: **20.05.2009**
(21) Application number: 07792491.8
(22) Date of filing: 14.08.2007
(51) Int. Cl.: A61K 35/74, A61P 3/06, A61P 9/10

(54) **LIPID-METABOLISM-AMELIORATING AGENT**

(30) Priority: 21.08.2006 JP 2006224672
(71) Applicant: Calpis Co., Ltd., Shibuya-ku, Tokyo 150-0022 (JP)
(72) Inventor: SUZUKI, Hiromi, Kanagawa 229-0006 (JP); FUJIWARA, Shigeru, Kanagawa 229- 0006 (JP)
(74) Representative: Nemec, Harald
(86) International application number: PCT/JP2007/065848
(87) International publication number: WO 2008/023608

(57) **Abstract**

Ppharmaceutical agents and food products which have no side effects, are easy to ingest continuously, and have a serum triglyceride-lowering effect are provided. A lipid metabolism-ameliorating agent and a serum triglyceride-lowering agent, each comprising as an active ingredient a culture of a bacterium belonging to the genus *Bacillus*, are disclosed. The bacterium belonging to the genus *Bacillus* is preferably *Bacillus subtilis*, and more preferably *Bacillus subtilis* C-3102 (FERM BP-1096). The lipid metabolism-ameliorating agent and the serum triglyceride-lowering agent are useful for treating and preventing diseases such as arteriosclerosis.

SEQUENCE LISTING

<110> Calpis Co. Ltd.

<120> LIPID-METABOLISM-AMELIORATING AGENT

<130> PCP-9006WO

<150> JP2006-224672
<151> 2006-08-21

<160> 2

<170> PatentIn version 3.1

<210> 1
<211> 30
<212> DNA
<213> Bacillus subtilis

<400> 1
gccccgcaca tacgaaaaga ctggctgaaa 30

<210> 2
<211> 30
<212> DNA
<213> Bacillus subtilis

<400> 2
ggatcccacg ttgtgattaa aagcagcgat 30

## Description

### TECHNICAL FIELD

The present invention relates to a lipid metabolism-ameliorating agent which comprises as an active ingredient cells or a culture of a bacterium of the genus *Bacillus*, particularly *Bacillus subtilis* C-3102, and which has a serum triglyceride-lowering action.

### BACKGROUND

An elevated serum triglyceride level is a major factor in the onset of diseases such as hyperlipidemia, arteriosclerosis, ischemic heart disease (angina pectoris and myocardial infarction), and diabetes, and is generally an indicator of lifestyle diseases. Moreover, it is known that a postprandial rise in neutral fat level and the persisting of higher level is the greatest factor influencing cardiovascular disease.

Pharmacotherapy and dietary therapy which restricts lipid intake are generally selected for treating hyperlipidemia. Drugs such as HMG-CoA reductase inhibitors (e.g., Mevastatin), which inhibit cholesterol biosynthesis by inhibiting the synthesis of valonic [sic] acid from 3-hydroxy-3-methylglutaryl cofactor A (HMG-CoA) by the cholesterol synthesis pathway, and bile acid adsorbers (anion exchange resins) are used in pharmacotherapy.

However, HMG-CoA reductase inhibitors have side effects, such as anaphylaxis, liver function abnormalities and rhabdomyolysis. Also, when anion exchange resins such as cholestyramine are used, the anion exchange resin adsorbs the bile acids which contain much cholesterol within the digestive tract, thereby blocking enterohepatic cycling and causing the cholesterol to be excreted from the body. However, anion exchange resins are difficult to swallow in a large dose. In addition, they may cause constipation and digestive tract symptoms. As for dietary therapy, this approach is accompanied by mental distress owing to dietary restrictions, making it difficult to continue over an extended period of time.

Accordingly, there exists a desire for drugs and food products with a serum triglyceride-lowering activity which have no side effects and is easy to ingest for a long time.

### DISCLOSURE OF THE INVENTION

The object of the invention is to provide a lipid metabolism-ameliorating useful for treating and preventing diseases such as arteriosclerosis.

The present invention provides a lipid metabolism-ameliorating agent comprising as an active ingredient cells or a culture of a bacterium belonging to the genus *Bacillus*. The invention also provides a serum triglyceride-lowering agent comprising as an active ingredient cells or a culture of a bacterium belonging to the genus *Bacillus*. The bacterium belonging to the genus *Bacillus* is preferably *Bacillus subtilis*, and more preferably *Bacillus subtilis* C-3102 (FERM BP-1096).

### BRIEF DESCRIPTION OF THE DRAWINGS

FIG. 1 shows the restriction enzyme NotI or SfiI digestion patterns of genomic DNA from *Bacillus subtilis* C-3102.
FIG. 2 shows the change over time in serum triglyceride concentration in subjects who ingested the lipid metabolism-ameliorating agent of the present invention.

### PREFERRED EMBODIMENTS OF THE INVENTION

Bacteria of the genus *Bacillus* (e.g., *Bacillus subtilis*) have long been closely associated with the dietary habits of man. Although ample information exists on the functionality of this organism, it has not previously been reported to have a serum triglyceride-lowering action or a lipid metabolism-ameliorating effect.

The lipid metabolism-ameliorating agent and serum triglyceride-lowering agent of the present invention are characterized by comprising as an active ingredient cells or a culture of a bacterium of the genus *Bacillus*, and preferably cells or a culture of *Bacillus subtilis*. The bacteriological characteristics of *Bacillus subtilis* are described in, for example, Bergey's Manual of Bacteriology, Vol. 11 (1986), and include the following.
(1) Gram positive
(2) Forms oval spores
(3) Rod-shaped
(4) Motile
(5) Aerobic
(6) Catalase: positive
(7) Growth at 50°C: +
(8) Growth at pH 5.7: +
(9) Utilization of citrate: +
(10) Acid production from the sugars arabinose, glucose, xylose, mannitol: +
(11) VP reaction: +
(12) Starch hydrolysis: +
(13) Nitrate reduction: +
(14) Indole production: -
(15) Gelatin hydrolysis: +
(16) Casein hydrolysis: +
(17) Film formation in liquid medium: +
(18) Curdling of milk: -
(19) Peptonization of milk: +

The *Bacillus subtilis* used in the lipid metabolism-ameliorating agent and serum triglyceride-lowering agent of the invention may include, for example, *Bacillus subtilis* C-3102 (deposited on December 25, 1985 at Japan's National Institute of Bioscience and Human Technology under accession number FERM BP-1096). Soybean cultures of *Bacillus subtilis* C-3102 have a number of desirable effects in livestock, such as improving the intestinal flora, somatic growth, preventing infection, increasing eggshell strength, enhancing meat quality and ameliorating fecal odors, and are used as feed additives (Japanese Patent Publication No. H4-24022). The beneficial effects of this strain on human health include regulating intestinal function and decreasing the products of intestinal putrefaction (Chonai Saikin Gakkaishi (Journal of Intestinal Microbiology) Vol. 18, No. 2, 93-99 (2004)).

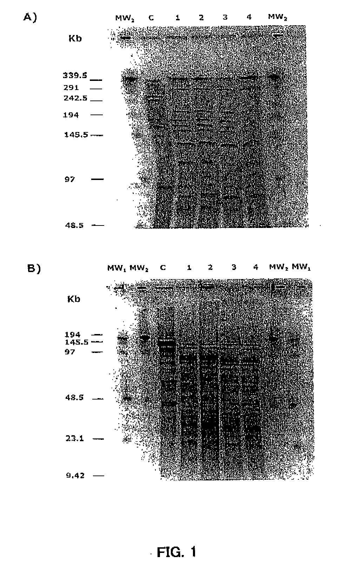

With *Bacillus subtilis* C-3102, an approximately 700 bps fragment is amplified by PCR using the PCR primers of SEQ ID NOs:1 and 2 below. In other *Bacillus subtilis* strains, no amplification is observed with these PCR primers. The approximately 700 bps fragment amplified with the *Bacillus subtilis* C-3102 genome as the template has no homology with the amylase sequence. This clearly distinguishes the C-3102 strain from other *Bacillus subtilis* strains.
Sequence 1: 5'-GCCCCGCACATACGAAAAGACTGGCTGAAA-3' (SEQ ID NO: 1)
Sequence 2: 5'-GGATCCCACGTTGTGATTAAAAGCAGCGAT-3' (SEQ ID NO: 2)
*Bacillus subtilis* C-3102 also has the following characteristics.
(1) Lacks plasmid DNA.
(2) The digestion pattern obtained from digestion of the genomic DNA with the restriction enzyme NotI or SfiI, and separation by agarose electrophoresis is as shown in FIG. 1.
(3) Produces antibacterial substances to B. cerous.
(4) Lacks resistance to ampicillin, chloramphenicol, ciprofloxacin, erythromycin, gentamicin, kanamycin, linezolid, quinupristin/dalfopristin, rifampin, streptomycin, tetracycline, trimethoprim and vancomycin (in each case, the minimum inhibitory concentration is from 0.03 to 4 pg/mL).

*Bacillus subtilis* may be cultured using a liquid or solid culture medium commonly used to grow microorganisms, which contains, for example, a carbon source, a nitrogen source and inorganic substances. The carbon source may be any of those can be utilized by *Bacillus subtilis*, such as glucose, fructose, sucrose, starch or molasses. Examples of nitrogen sources that may be used include peptones, casein hydrolyzates, meat extracts, and ammonium sulfate. In addition, phosphoric acids and salts thereof, such as potassium, magnesium, calcium, sodium, iron and manganese salts, and also vitamins, amino acids and surfactants may be added as needed. In addition to such synthetic media. *Bacillus subtilis* may also be cultured using substances derived from natural products, such as soybean oil meal. Cultivation is preferably carried out under aerobic conditions. Preferred examples of the culture apparatus include apparatuses for aeration spinner liquid culturing with a jar fermentor, tray-type solid culture apparatuses, and automated *koji*-making culture apparatuses. The culture temperature is preferably from 20 to 50°C, and more preferably from 30 to 45°C; the culture period is from 12 hours to 7 days; and the initial pH is preferably from 5 to 9, and more preferably from 6 to 8.

The culture thus obtained contains *Bacillus subtilis* cells, medium and fermentation products. The culture may be used directly without processing as a lipid metabolism-ameliorating agent or a triglyceride-lowering agent. Alternatively, the culture may be concentrated and used. Excipients and other ingredients may be added to the culture or the concentrated culture for use as a preparation in the form of dry powder, granules or tablets. Cells isolated from the culture, the culture free from the cells, or the culture containing the cells may be used in the present invention. In an especially preferred embodiment. *Bacillus subtilis* is cultured using substances of natural origin which are suitable for use as foods, such as soybean oil meal, boiled soybeans, boiled adzuki beans, boiled rice, rice boiled with barley, wheat bran, boiled corn, and other grains, and is directly mixed into a food product without separating the cells from the culture.

The lipid metabolism-ameliorating agent and serum triglyceride-lowering agent of the invention may be taken in the form of, for example, a liquid, powder, granules or tablets, or may be mixed as a food additive into beverage and food products and ingested. Illustrative examples of beverage and food products include drinks, candies and sweets, pastes, bread, processed fish and meat products, and dairy products. The lipid metabolism-ameliorating agent and serum triglyceride-lowering agent of the invention may be added to these various food materials and furnished as health drinks, health foods, or nutraceuticals having health-promoting benefits.

As described above, the present invention provides a lipid metabolism-ameliorating agent with a serum triglyceride-lowering activity, which comprises as an active ingredient *Bacillus subtilis* cells or a culture thereof. The *Bacillus subtilis* serving as the active ingredient in the present invention is effective in very small amounts and in a short period of time. It has an excellent preservability and acid resistance, easily reaches to and grows within the large intestine, and can be expected to have a sustained serum triglyceride-lowering effect.

The entire contents of all patents and reference documents cited in this specification are incorporated herein by reference. Also, the entire contents of the specification and drawings of Japanese Patent Application No. 2006-224672, which serves as the basis for the priority claim of the present application, are incorporated herein by reference.

The present invention is described in more detail below by way of a working example, although the scope of the invention is not limited by the examples.

### Example 1

In the following working example, *Bacillus subtilis* C-3102 (deposited on December 25, 1985 at Japan's National Institute of Bioscience and Human Technology under accession number FERM BP-1096) was used as an example of a bacterium belonging to the genus *Bacillus*.

Five kilograms of tap water was added to 5 kg of granulated commercial soybean oil meal, and the mixture was sterilized at 121°C for 120 minutes, then inoculated with a culture broth of *Bacillus subtilis* strain C-3102 that had been pre-cultured. The resulting mixture was cultured at 37°C for 40 hours to produce a soybean culture of *Bacillus subtilis* C-3102. The resulting culture was dry ground, blended with other ingredients shown in the table below, and 500 mg tablets (each containing 3×10⁹ *Bacillus subtilis* spores) were prepared. Table 1-1 shows the nutrients contained within the tablets, and Table 1-2 shows the composition of the tablets.

**Table 1**

| Nutrient analysis of tablets (per 100 g) | | |
|---|---|---|
| Nutrients | | Values |
| Protein | g/100 g | 4.1 |
| Lipids | g/100 g | 1.4 |
| Ash | g/100 g | 18 |
| Carbohydrates | g/100 g | 74.6 |
| Energy | Kcal/100 g | 327 |
| Sodium | Mg/100 g | 20.2 |

### Tablet composition

| Ingredient | Content (%) |
|---|---|
| Powdered sugar | 64.10% |
| Eggshell calcium | 22.00% |
| Glucose | 6.00% |
| *Bacillus subtilis* C-3102 soybean culture | 5.60% |
| Sucrose ester | 1.00% |
| Shellac | 0.60% |
| Gum arabic powder | 0.35% |
| Carnauba wax | 0.35% |

### Example 2

Serum Triglyceride Rise Suppression Test (Human Ingestion Test)

### Procedure:

Ten healthy males and females (5 women and 5 men) from 25 to 40 years of age were selected as the subjects. The selection criteria are shown in Table 2. Individuals taking or ingesting specific drugs or foods for specified health use were excluded.

**Table 2**

| Ten individuals (5 men and 5 women) who satisfied the following criteria and did not meet the exclusion criteria were chosen as subjects. |
|---|
| Selection criteria (criteria for selecting the subjects) |
| (1) Individuals from 25 to 40 years of age. |
| (2) Individuals not on any medication and free of apparent illness (TG. T-cho, BS, blood pressure, constipation, etc.). |
| a) Neutral fat < 250 mg/dL. |
| b) Total cholesterol < 240 mg/dL. |
| c) Fasting blood sugar < 126 mg/dL. |
| d) GOT < 50 IU/L; GPT < 50 IU/L; γ-GTP < 100 IU/L. |
| e) Systolic blood pressure < 140 mmHg; Diastolic blood pressure < 90 mmHg. |
| (3) Individuals with a relatively constant diet and level of exercise. |
| (4) Individuals receiving regular examinations at a medical facility. |
| (5) Individuals who do not manifest an anaphylactic reaction to ingestion of the food under study (fermented soybean culture). |
| (6) Individuals capable of limiting their intake of alcoholic beverages (to not more than the equivalent of 500 mL of beer per day). |
| (7) Individuals able to maintain a constant daily lifestyle during the course of the test. |
| (8) Individuals able to keep a diary (to record ingestion of the agent under study, exercise, diet and body weight) throughout the period of the test. |
| (9) Individuals who, prior to the start of the test, have given their consent to participate in the test and have impressed their personal seal to or signed the consent form and entered the date. |

The subjects orally ingested one 500 mg tablet (a *Bacillus subtilis* C-3102 soybean culture tablet containing 3×10⁹ spores) daily, whenever possible after breakfast, for
a period of four weeks. Fasting blood samples (no eating or drinking after 9 PM the night before; blood drawn at a fixed time in the morning) were collected before the start of ingestion and after one week, two weeks, three weeks and four weeks from the start of ingestion, and the serum triglyceride level in each sample was measured. The test schedule is shown in Table 3.

**Table 3 Test schedule (test items/period)**

| | Period of ingestion (4 weeks) | | | | |
|---|---|---|---|---|---|
| Test Items/Period (week) | Start | 1 week | 2 weeks | 3 weeks | 4 weeks |
| | around Sep 22 | around Sep 29 | around Oct 6 | around Oct 13 | around Oct 20 |
| Body Mass Index + Cardiovascular Exam Height (initial exam), weight, body fat percentage (BI method), blood pressure, pulse, temperature | ○ | ○ | ○ | ○ | ○ |
| Blood Biochemistry (1) TG, T-cho, AG ratio, total bilirubin, fasting blood sugar, GOT, GPT, AL-P, γ-GTP, amylase, LDL-cho, HDL-cho, LDH, total protein, albumin, UA, BUN, creatinine, ZTT, Na, Cl, K, Ca, ionized calcium, P, Mg, Fe | ○ | ○ | ○ | ○ | ○ |
| Blood Biochemistry (2) PT (prothrombin), hepaplastin, HbAlc, TT (Thrombotest), fibrinogen, APTT (thromboplastin time), vitamin K fraction | ○ | ○ | ○ | ○ | ○ |
| General Blood Tests WBC, RBC, Hb, Ht, MCV, MCH, MCHC, platelets | ○ | ○ | ○ | ○ | ○ |
| General Urine Tests Sugars, protein, urobilinogen, sediment (performed when protein-positive) | ○ | ○ | ○ | ○ | ○ |
| Patient Interview by Doctor: Examination Checked for subjective symptoms and adverse events, checked daily journal and provided guidance | ○ | ○ | ○ | ○ | ○ |

| | | | | | |
|---|---|---|---|---|---|
| ○: tested | | | | | |

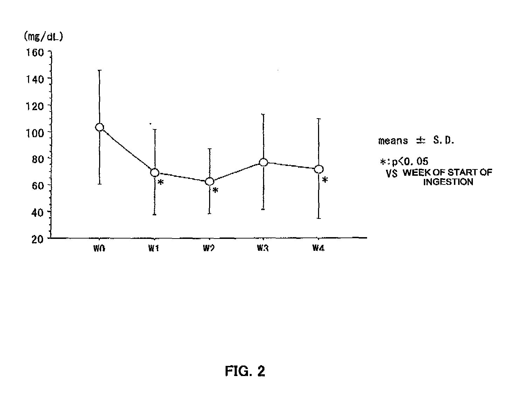

The results of the above test are shown in FIG. 2. The serum triglyceride level over time was 103.6±42.82 mg/dL at the start of ingestion, 69.6±32.00 mg/dL after one week of ingestion, 63.0±24.58 mg/dL after two weeks of ingestion, 77.0±35.63 mg/dL after three weeks of ingestion, and 71.8±37.26 mg/dL after four weeks of ingestion. A one-way analysis of variance for repeated measurements based on the ingestion period revealed a significant difference (p<0.01). As a result of multiple comparison (Tukey-Kramer test), significant differences were observed between the start of ingestion and after one week of ingestion (p<0.05), between the start of ingestion and after two weeks of ingestion (p<0.05), and between the start of ingestion and after four weeks of ingestion (p<0.05). From the above results, it was apparent that soybean cultures of *Bacillus subtilis* C-3102 have a serum triglyceride-lowering effect.

### INDUSTRIAL APPLICABILITY

The lipid metabolism-ameliorating agent of the present invention is able to lower the serum triglyceride concentration in humans, and is thus useful as an agent for preventing and ameliorating arteriosclerosis.

## Claims

1. A lipid metabolism-ameliorating agent comprising as an active ingredient a culture of a bacterium belonging to the genus *Bacillus*.

2. The lipid metabolism-ameliorating agent according to claim 1. wherein the bacterium belonging to the genus *Bacillus* is *Bacillus subtilis*.

3. The lipid metabolism-ameliorating agent according to claim 1 or 2, wherein the bacterium belonging to the genus *Bacillus* is *Bacillus subtilis* C-3102 (FERM BP-1096).

4. A serum triglyceride-lowering agent comprising as an active ingredient a culture of a bacterium belonging to the genus *Bacillus*.

5. The serum triglyceride-lowering agent according to claim 4, wherein the bacterium belonging to the genus *Bacillus* is *Bacillus subtilis*.

6. The serum triglyceride-lowering agent according to claim 4 or 5, wherein the bacterium belonging to the genus *Bacillus* is *Bacillus subtilis* C-3102 (FERM BP-1096).

7. A lipid metabolism-ameliorating agent comprising as an active ingredient a bacterium belonging to the genus *Bacillus*.

8. A lipid metabolism-ameliorating agent comprising as an active ingredient a bacterium belonging to *Bacillus subtilis*.

9. A lipid metabolism-ameliorating agent comprising as an active ingredient *Bacillus subtilis* C-3102 (FERM BP-1096).

10. A serum triglyceride-lowering agent comprising as an active ingredient a bacterium belonging to the genus *Bacillus*.

11. A serum triglyceride-lowering agent comprising as an active ingredient a bacterium belonging to *Bacillus subtilis*.

12. A serum triglyceride-lowering agent comprising as an active ingredient *Bacillus subtilis* C-3102 (FERM BP-1096).
